Europäisches Patentamt

European Patent Office

Office européen des brevets

(11) Veröffentlichungsnummer: **0 355 795 B1**

(19)

## (12) EUROPÄISCHE PATENTSCHRIFT

(45) Veröffentlichungstag der Patentschrift: **23.12.92**

(21) Anmeldenummer: **89115471.8**

(22) Anmeldetag: **22.08.89**

(51) Int. Cl.⁵: **A61J  1/00**

(54) **Adapter zum Anschliessen von enteralen Überleitungsgeräten.**

(30) Priorität: **24.08.88 DE 3828729**

(43) Veröffentlichungstag der Anmeldung:
**28.02.90 Patentblatt  90/09**

(45) Bekanntmachung des Hinweises auf die
Patenterteilung:
**23.12.92 Patentblatt  92/52**

(84) Benannte Vertragsstaaten:
**AT BE DE ES FR GB IT NL SE**

(56) Entgegenhaltungen:
**CH-A- 350 423          DE-B- 1 039 193**
**DE-U- 8 703 587          GB-A- 778 794**
**US-A- 1 366 789          US-A- 3 467 270**

(73) Patentinhaber: **N.V. Nutricia**
**Stationsstraat 186**
**NL-2712 HM Zoetermeer(NL)**

(72) Erfinder: **Iwatschenko, Peter**
**Bürgerholzweg 4**
**W-8524 Neunkirchen(DE)**
Erfinder: **Prell, Walter**
**Brunneweg 1**
**W-8551 Hallerndorf(DE)**
Erfinder: **Wolkenstörfer, Reinhold**
**Anna-Friedrich-Strasse 5a**
**W-8524 Neunkirchen(DE)**

(74) Vertreter: **von Hellfeld, Axel, Dr. Dipl.-Phys. et**
**al**
**Wuesthoff & Wuesthoff Patent- und Rechts-**
**anwälte Schweigerstrasse 2**
**W-8000 München 90(DE)**

EP 0 355 795 B1

**Beschreibung**

Die Erfindung betrifft einen Adapter zum wahlweisen Anschließen von enteralen Überleitungsgeräten an jeweils eine von verschiedenen Flaschen, mit den Merkmalen des Oberbegriffs des Patentanspruchs 1.

Ein solcher Adapter ist aus der US-A-1 366 789 bekannt. Die dort vorgesehenen, unterschiedlichen Kappen sind fest miteinander verbunden, sodaß sie sich nur gemeinsam drehen lassen.

Auch aus der DE-U-8 703 587 ist ein ähnlicher Adapter bekannt.

Bei der enteralen Ernährung wird einem Patienten flüssige Nahrung durch eine Sonde zugeführt, die mit einem Überleitungsgerät verbunden ist, das seinerseits mit einer die Nahrung enthaltenden Flasche in Verbindung steht.

Bei der enteralen Ernährung werden unterschiedliche, insbesondere normierte Flaschentypen verwendet, die wegen ihrer unterschiedlich geformten Flaschenhälse auch unterschiedliche Adapter erfordern. Sollen deshalb unterschiedliche Flaschen auf dem enteralen Überleitungsgerät montiert werden, so sind jeweils angepaßte Adapter erforderlich. Dadurch, daß jeweils auf den verwendeten Flaschentyp abgestimmte Adapter erforderlich sind, werden die Kosten erhöht und es ist eines aufwendige Vorratshaltung im Krankenhaus erforderlich.

Aus der US-A-3467270 ist ein Adapter bekannt, an den unterschiedliche Flaschen angeschlossen werden können. Bei diesem bekannten Adapter sind aber zwei Kappen mit unterschiedlichen Durchmesser einstückig ausgebildet, so daß sie nicht relativ zueinander verdrehbar sind und auch keine Flaschenwandung zwischen die Kappen paßt. Aus der DE-B-1039193 ist ein Verschluß zum gleichzeitigen Verschließen von zwei Flaschen bekannt, deren Inhalte gemischt werden sollen. Diese Druckschrift betrifft somit keinen Adapter zum Anschließen von Flaschen, sondern das Gegenteil, nämlich eine Vorrichtung zum Verschließen von Flaschen.

Der Erfindung liegt die Aufgabe zugrunde, den Eingangs genannten Adapter so zu verbessern, daß die Handhabbarkeit bei guter Dichtwirkung verbessert ist.

Die erfindungsgemäße Lösung dieser Aufgabe ist im Patentanspruch 1 gekennzeichnet. Bevorzugte Ausgestaltungen des erfindungsgemäßen Adapters sind in den Unteransprüchen beschrieben.

Nachfolgend wird ein Ausführungsbeispiel der Erfindung anhand der Zeichnung naher erläutert. Es zeigt:

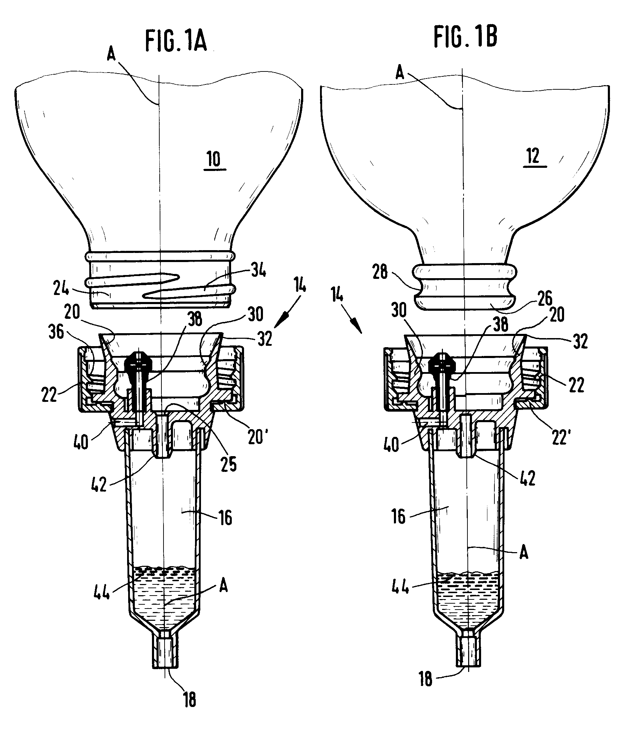

Fig. 1A     einen erfindungsgemäßen Adapter mit einer Flasche, und

Fig. 1B     den gleichen Adapter wie in Fig. 1A mit einer im Vergleich einen engeren Hals aufweisenden Flasche.

In den Fig. 1A und 1B sind zwei Flaschen 10, 12 dargestellt, die sich dadurch unterscheiden, daß ihre Öffnungshälse unterschiedlich sind. Es wird aber jeweils der gleiche Adapter 14 verwendet, um die Flaschen 10 oder 12 mit einer Einrichtung zu verbinden, über welche in den Flaschen enthaltene Flüssigkeit einem Patienten zugeführt wird. Beim dargestellten Ausführungsbeispiel ist der Adapter 14 mit einer Tropfkammer 16 verbunden, die als solche dem Fachmann bekannt ist und hier nicht näher beschrieben zu werden braucht.

Die Tropfkammer 16 ist über eine Leitung 18 mit dem Patienten verbunden.

Der Adapter 14 weist eine innere Kappe 20 und eine äußere Kappe 22 auf. Die innere Kappe 20 dient dem Anschluß der Flasche 12 mit engerem Flaschenhals, während die äußere Kappe 22 dem Anschluß einer Flasche 10 mit (im Vergleich zur Flasche 12) weiterem Flaschenhals 24 dient. Beide Kappen haben unten einen Durchlaß 25.

Der engere Flaschenhals 26 weist eine umlaufende Vertiefung 28 auf, in die ein Wulst 30 der inneren Kappe 20 eingreift. Die innere Kappe 20 ist also als elastischer Schnappverschluß ausgebildet.

Die Flasche 10 mit weiterem Flaschenhals 26 ist mit einem Aussengewinde 34 versehen, das mit einem Innengewinde in der äusseren Kappe 22 zusammenwirkt. Die äußere Kappe 22 ist also als Überwurfmutter ausgebildet. Wird die äußere Kappe 22 gemäß Fig. 1A auf das Außengewinde 34 des Flaschenhalses 24 der Flasche aufgeschraubt, so schmiegt sich eine Dichtlippe 32 der inneren Kappe 20 innenseitig dicht an die Flaschenwand an, so daß die Flüssigkeit aus der Flasche 10 nicht in den Totraum zwischen den beiden Kappen gelangen kann, sondern ausschließlich über ein Tropfrohr 42 in die Tropfkammer 16 strömt.

Beim in Fig. 1 dargestellten Ausführungsbeispiel ist die innere Kappe 20 mit einem nach außen ragenden Flansch 20' versehen, hinter den ein nach innen ragender Flansch 22' der äußeren Kappe 22 greift, so daß bei auf die Flasche 10 aufmontierter äußerer Kappe 22 eine insgesamt dichte Preßverbindung entsteht.

Die Flaschen 10, 12, die innere Kappe 20 und die äußere Kappe 22, das Tropfrohr 42 sowie die Tropfkammer 16 sind koaxial in bezug auf eine gemeinsame Achse A ausgerichtet. Die durch das Tropfrohr 42 tropfende Flüssigkeit (z.B. Milch) bildet in der Tropfkammer 16 in bekannter Weise einen Flüssigkeitsspiegel 44.

Beim dargestellten Ausführungsbeispiel sind die innere und die äußere Kappe getrennt aus Kunststoff hergestellt. Ein Belüftungsventil 38 ist in der inneren Kappe 22 ausgebildet und ermöglicht, daß über einen Kanal 40 Luft von außen in die Flaschen eintreten kann. Das Belüftungsventil 38 ist als sogenanntes Einwegventil ausgebildet, d.h. es kann nur Luft von außen über den Kanal 40 nach innen strömen, hingegen keine Flüssigkeit über den Kanal 40 nach außen austreten.

## Patentansprüche

1. Adapter zum wahlweisen Anschließen von enteralen Überleitungsgeräten an jeweils eine von verschiedenen Flaschen (10, 12), deren Hälse (24, 26) einen kleineren bzw. einen größeren Öffnungsdurchmesser aufweisen, mit zumindest zwei Kappen (20, 22), die koaxial und mit ihren Öffnungen in gleicher Richtung weisend angeordnet sind sowie verschiedene Öffnungsdurchmesser aufweisen,
dadurch **gekennzeichnet,** daß eine der Kappen (22) in bezug auf die andere Kappe (20) um eine gemeinsame Achse (A) der Kappen drehbar ist, wobei die andere Kappe (20) keine Drehung ausführt.

2. Adapter nach Anspruch 1,
dadurch **gekennzeichnet,** daß eine innere Kappe (20) von einer äußeren Kappe (22) einen radialen Abstand aufweist, der zumindest der Stärke einer Flaschenwand entspricht.

3. Adapter nach Anspruch 1 oder 2,
dadurch **gekennzeichnet,** daß die innere Kappe (20) als Schnappkappe ausgebildet ist.

4. Adapter nach einem der vorhergehenden Ansprüche,
dadurch **gekennzeichnet,** daß die äußere Kappe (22) als Überwurfmutter ausgebildet ist.

5. Adapter nach einem der vorhergehenden Ansprüche,
dadurch **gekennzeichnet,** daß in der inneren Kappe (20) ein Belüftungsventil (38) vorgesehen ist.

6. Adapter nach einem der vorhergehenden Ansprüche,
dadurch **gekennzeichnet,** daß die innere Kappe eine Dichtlippe (32) aufweist, die sich innenseitig an eine Flaschenwand anschmiegt.

7. Adapter nach einem der vorhergehenden Ansprüche,
dadurch **gekennzeichnet,** daß die Kappen (20, 22) jeweis sich in bezug auf die gemeinsame Achse (A) radial erstreckende Flansche (20' bzw. 22') aufweisen, die bei auf eine Flasche (10) montiertem Adapter eine dichte Preßverbindung bilden.

## Claims

1. An adapter for the selective connection of enteral transfer appliances to one each of different bottles (10, 12) whose necks (24, 26) have smaller or greater opening diameters, comprising at least two caps (20, 22) which are disposed coaxially, oriented in the same direction, and have different opening diameters, **characterized** in that one of the caps (22) is rotatable with respect to the other one (20) about a common axis (A) of the caps, while the other cap (20) does not carry out any rotation.

2. The adapter as claimed in claim 1, characterized in that an inner cap (20) is disposed radially spaced from an outer cap (22) at a distance which corresponds at least to the thickness of a bottle wall.

3. The adapter as claimed in claim 1 or 2, characterized in that the inner cap (20) is embodied by a snap-fit cap.

4. The adapter as claimed in any one of the preceding claims, characterized in that the outer cap (22) is embodied by a screw cap.

5. The adapter as claimed in any one of the preceding claims, characterized in that an air vent valve (38) is provided in the inner cap (20).

6. The adapter as claimed in any one of the preceding claims, characterized in that the inner cap comprises a sealing lip (32) the inside of which comes to lie snugly against a bottle wall.

7. The adapter as claimed in any one of the preceding claims, characterized in that the caps (20, 22) each comprise flanges (20' and 22', respectively) which extend radially with respect to the common axis (A) and establish a tight press-fit connection when the adapter is mounted on a bottle (10).

## Revendications

1. Adaptateur destiné à raccorder au choix des appareils de transition "intestinale" à chacune des différentes bouteilles (10, 12), dont les cols (24, 26) présentent un diamètre d'entrée assez petit ou assez grand, avec au moins deux calottes (20, 22) disposées coaxialement et ayant leurs ouvertures dirigées dans la même direction et présentant également des diamètres d'ouverture différents ,

**caractérisé** en ce qu'une des calottes (22) peut tourner par rapport à l'autre calotte (20) autour d'un axe commun (A) des calottes, l'autre calotte (20) n'effectuant aucune rotation.

2. Adaptateur selon la revendication 1, **caractérisé**, en ce qu'une calotte intérieure (20) présente une distance radiale par rapport à une calotte extérieure (22) correspondant au moins à l'épaisseur d'une paroi de bouteille.

3. Adaptateur selon la revendication 1 ou 2, **caractérisé** en ce que la calotte inférieure (20) a la forme d'une calotte encliquetable.

4. Adaptateur selon l'une des revendications précédentes, **caractérisé** en ce que la calotte extérieure (22) a la forme d'un écrou enveloppant.

5. Adaptateur selon l'une des revendications précédentes, **caractérisé** en ce qu'une soupape d'aération (38) est prévue dans la calotte intérieure (20).

6. Adaptateur selon l'une des revendications précédentes, **caractérisé** en ce que la calotte intérieure présente une lèvre d'étanchéité (32) qui s'applique du côté intérieur sur la paroi d'une bouteille.

7. Adaptateur selon l'une des revendications précédentes, **caractérisé** en ce que les calottes (20, 22) comportent chacune des brides (20' et 22') disposées radialement par rapport à l'axe commun (A), qui constituent un assemblage sous pression étanche pour l'adaptateur monté sur une bouteille (10)

FIG.1A

FIG.1B